# EUROPEAN PATENT APPLICATION

(11) **EP 2 679 503 A2**
(43) Date of publication of application: **01.01.2014**
(21) Application number: 13168359.1
(22) Date of filing: 17.05.2013
(51) Int. Cl.: B64F 5/00, B64D 11/00

(54) **Configuration and monitoring system for an aircraft cabin element, fuselage and aircraft comprising said system and method of monitoring an aircraft**

(30) Priority: 28.06.2012 US 201261665486 P
(71) Applicant: INTERTECHNIQUE, 78373 Plaisir Cedex (FR)
(72) Inventor: Boomgaarden, Günter, 23684 Scharbeutz (DE); Glück, Michael, 78373 PLAISIR CEDEX (FR)
(74) Representative: Eisenführ Speiser

(57) **Abstract**

The invention relates to a method and a configuration and monitoring system for an aircraft cabin element of a plurality of cabin elements in a cabin of an aircraft, the system comprising:
- a radio frequency identification (RFID) device, wherein the RFID-device is associated with the aircraft cabin element adapted for providing a monitor signal adapted to indicate a status of the aircraft cabin element by a monitor information,
- a RFID-reader communicatingly connectable to the RFID-device,
- a controlling unit of a central cabin system. According to the invention
- the RFID-reader is communicatingly integrated in the central cabin system by being continuously communicatingly connectable to the controlling unit in the cabin, wherein information are centrally collectable and accessable by the controlling unit, and
- at least one repeater is adapted for expanding the monitor signal in the cabin to the RFID-reader, wherein the monitor information includes continuously, responding status data of the aircraft cabin element during the flight.

## Description

The invention relates to a configuration and monitoring system for an aircraft cabin element of a plurality of cabin elements in a cabin of an aircraft according to the preamble part of claim 1. Further the invention relates to a fuselage and an aircraft comprising a configuration and monitoring system for an aircraft cabin element and a method of monitoring an aircraft.

The configuration and monitoring of an aircraft prove to be complex, laboriously and therefore costly for the whole aircraft as well as the parts of the aircraft like the cockpit, turbine engine or fuselage. Also each part has a very complex built up and is divided in smaller parts like in the rear of the fuselage, also known as a cabin, featured for passengers. A most important feature is the breathable atmosphere for a passenger and a crew member because of the air pressure at cruising altitudes. At cruising altitudes of a amodern commercial aircraft the surrounding atmosphere is too thin for passengers and crew to breathe without an oxygen mask; so cabins are pressurized at a higher pressure than ambient pressure at altitude. In a commercial air travel, particularly in airliners, cabins may be divided into several parts. For passengers it is sectioned in a travel class section in medium and large aircrafts, areas for flight attendants, the galley and storage for inflight service. Seats are mostly arranged in rows and alleys. There are plenty of supply items available for providing comfort and safety aspects for the passenger of each section mostly adapted by devices and component assemblies.

A very know supply item is the passenger service unit, wherein a reading light, an attendant call button, oxygen masks, inflatable life jacket, ventilation nozzles, loudspeaker are integrated for providing comfort to the passenger seat; this in addition to generally provided functions like control of temperature, air pressure, content of oxygen, the flight information, installed entertainment or the air/ground connectivity.

The control of the supply items, but also the maintenance of the items and components assemblies and the components themselves require a high operating expense of recording and controlling. The tracking of the status of maintaining becomes even more complicated after certain maintenance procedures wherein only some of the components or elements will be replaced, so that the life time of each part component or even of one element will differs and leading to a high volume of data not able to manage without occurred errors or occurred wrong information.

Barcodes radio-frequency identification (RFID) technology can be provided to track and manage inventory, assets, people, etc. For example, it can be affixed to cars, computer equipment, books, mobile phones, etc. This technology is a powerful and effective technology typically using the wireless non-contact use of radio-frequency electromagnetic fields to transfer data, for the purposes of automatically identifying and tracking tags attached to objects. The tags contain stored information in a non-volatile memory. Corresponding to unique tag serial number, a product-related information such as a stock number, lot or batch number, production date, or other specific information.

RFID tags usually contain at least two parts: an integrated circuit for storing and processing information, modulating and demodulating a radio-frequency (RF) signal, collecting DC power from the incident reader signal, and other specialized functions; and an antenna for receiving and transmitting the signal. An RFID-reader transmits an encoded radio signal to interrogate the tag. The tag receives the message and responds with its identification information. A difference is made in RFID between inductive and electromagnetic coupling, depending on the frequency range and leads to active or passive tags.

Unlike a bar code, the tag does not necessarily need to be within line of sight of the reader, and may be embedded in the tracked object. RFID tags are availed in a wide variety of shape and size so that tags can be conFig.d as small as possible de-pending of the element or component. RFID offers advantages over manual systems or use of bar codes. The tag can be read if passed near a reader, even if it is covered by the object or not visible being inside of a case, carton, box or other container. Tagging and tracking of products and devices using RFID is widely used in manufacturing and packaging processes.

US 2007/0114280 discloses an aircraft component and maintenance tracking system in the maintenance modus on ground of an aircraft. The system includes aircraft components, RFID tags and a transceiver being conFig.d to receive information transmitted by each of the RFID tags which are conFig.d to transmit identification and maintenance data information that is representative of a maintenance history for example manufacturing facility structures, aircraft inspection facility structures, hangar door, ramp surface, taxiway surfaces and so on.

Still, the aircraft component and maintenance tracking system of an aircraft have drawbacks as the limitation of the handling of a hand held reader and the mean time to restore during the maintenance status on the ground of the airport, the missing life history of each component and supply item, the missing callable data during flight.

This is where the invention comes in, a major object of the present invention is to provide an aircraft cabin element configuration and monitoring system for a cabin element from a plurality of cabin elements in a cabin of an aircraft which is adapted for improved monitoring. In particular it is an object to provide a system with improved safety aspects, in particular a system and method adapted to allow an actual status of maintenance to be available and/or to allow an actual status to be available instantly and/or to allow an actual status to be available instantly in the aircraft. It is still a further object to improve the monitoring of an aircraft with a technical realisation and technical implementation in the cabin, furthermore in the fuselage. in particular it is an object to provide an automatically monitoring of different status data of aircraft components for a cabin during the flight. It is still further an object of the invention to increase the reliability of supply items concerning safety and technician aspects with a permanently monitoring during flight for the cabin and the fuselage. It is still another object of the invention provide a method of monitoring an aircraft.

In relation to the configuration and monitoring system for an aircraft cabin element from a plurality of cabin elements in a cabin of an aircraft, the object of the invention is achieved by the configuration and monitoring system as mentioned in the introduction wherein according to the invention further the features of the characterizing part of claim 1 are provided. Thus, the invention relates to a configuration and monitoring system for an aircraft cabin element of a plurality of cabin elements in a cabin of an aircraft, the system comprising:
- a radio frequency identification device (RFID), wherein the RFID-device is associated with the aircraft cabin element adapted for providing a monitor signal adapted to indicate a status of the aircraft cabin element by a monitor information,
- a RFID-reader communicatingly connectable to the RFID-device,
- a controlling unit of the central cabin system.

In accordance with the invention the RFID-reader is communicatingly integrated in the central cabin system by being continuously communicatingly connectable to the controlling unit in the cabin, wherein information, in particular all information, are centrally collectable and accessable by the controlling unit. Further, according to the invention, at least one repeater is adapted for expanding the monitor signal in the cabin to the RFID-reader, wherein the monitor information includes continuously responding status data of the aircraft cabin element during the flight.

Thus basically, the invention provides an aircraft cabin element configuration and monitoring system wherein the ready is continuously communicatingly connectable to the controlling unit for receiving continuously responding status data of the aircraft cabin element during the flight. The repeater can expand the monitor signal in the cabin to the RFID-reader by one or more functions. E.g. the repeater can simply relay or route the monitor signal from another repeater to a further repeater or from the RFID-device to a further repeater or from the RFID-device to a the RFID-reader directly. In particular the repeater can simply transmit or amplify the monitor signal or, also, the repeater can be activated by the RFID-device and/or the RFID-reader. Preferably the repeater and/or the RFID-device and/or the RFID-reader are powered by an energy harvesting device, in particular are connected for powering to a an energy harvesting system.

In relation to the aircraft cabin element configuration and monitoring system for a cabin the invention also leads to a fuselage of an aircraft of claim 9. The invention also leads to an aircraft of claim 10. Thus, the configuration and monitoring functionality of the concept can also be provided to an aircraft cabin element and/or a fuselage element and/or an outside-cabin element alone or in combination.

As relates the method the object is achieved by the method as mentioned in the introduction. In accordance with the invention the method also comprises the steps of the characterizing part of claim 11.

The invention recognized that it is desirable to provide a configuration and monitoring system which is adapted for all the different types of cabin elements in a cabin of an aircraft continuously monitoring them like an online modus of monitoring during the flight. Thus, the invention provides an inventive concept of aircraft cabin element configuration and monitoring system for a cabin element comprising a radio frequency identification (RFID) device, a RFID-reader communicatingly connectable to the RFID-device, a controlling unit of the central cabin system, wherein the RFID-reader is communicatingly integrated in the central cabin system by being continuously communicatingly connectable to the controlling unit in the cabin. The RFID-device is associated with the aircraft cabin element adapted for providing a monitor signal adapted to indicate a status of the aircraft cabin element by monitor information. The monitor information of the invention is more than a storage information such as a programmed stock or batch number. The monitor information indicates the status meaning the continuously updating of the status of the device. The invention further provides systems wherein, preferably all, monitor information are collectable and accessable by the controlling unit. The inventive concept allows to replace an externally used hand held reader with an external RFID-database focused, in particular only focused, on the read out of the hand held readers. The invention also offers the opportunity to involve the crew for progressive interacting with the components and supply items on board during flight. The continuously responding status data also guarantee a detailed overview being independent of the time to time maintenance report on the ground.

According to the invention the RFID-reader is communicatingly integrated in the central cabin system by being continuously communicatingly connectable to the controlling unit in the cabin. The controlling unit, also sometime described as cabin management system, is used for controlling the function of the cabin and therefore its devices and supply modules. By integrating the RFID-reader in the controlling unit being continuously connected to the control unit the function of the control unit is increased and becomes a function as supervisory unit for the whole functions of the cabin of an aircraft.

The invention further recognized that it is desirable to get all information automatically independent of the location being placed in a cabin. The invention advantageously provides at least one repeater adapted for expanding the monitor signal in the cabin to the RFID-reader. For the installed monitoring system it must be guaranteed that all RFID-devices are callable by the RFID-reader for getting the responding status of each device. The repeater increases the communication ability between reader and RFID-device expanding the signal of the device and improves therefore the communication between the device and the reader adapted in a suitable operating distance. The integration of the repeater has a huge potential of repackaging a multifunctional RFID-system. This suitable operating distance also allows for far out devices the communication to the RFID-reader and replaces the laborious readout operated by the hand held reader for low energy RFID tags.

The invention further recognized that the handling of a hand held reader is disadvantageous and complicates the monitoring of a high sophisticated installation of a cabin; also impossible to manage during the flight. The invention provides a configuration and monitoring system for an aircraft cabin element from a plurality of cabin elements in a cabin of an aircraft wherein the hand out reader, discontinuously used by a person for getting signals, basically is replaced by an RFID-reader; preferably connected to a controlling unit getting continuously responding status data as a monitor signal.

These and the further developed configurations of the invention are further outlines in the dependent claims. Thereby, the mentioned advantages of the proposed concept are even more improved. For each feature of the dependent claims it is claimed independent protection independent of all other features of this disclosure.

In a particular preferred development it has been recognized to be convenience when the cabin element including the continuously, responding status data is adapted to a cabin component and/or a cabin supply items. This offers the opportunity for monitoring components and cabin supply item consisting of cabin elements with functionally use. Further this development provides an abstractly reduced collecting of the monitor like status data for a function and single function or the whole function of the cabin component and cabin supply item. In this case a single cabin element can failed without decreasing or only slight decreasing of the functionality of the component or supply item.

In a particular preferred development a transponder and a sensor unit is assigned to the RFID, in particular a sensor unit is integrated in the RFID, communicating continuously, responding status data providing analyzable and transponder callable status data. The supply item usually integrates analyzable or measurable values with a need for detecting the real value. In the state of art the detecting of analyzable values is solved by a sensor unit connect to the central cabin system. The advantageous step of this embodiment is the connecting of an RFD device integrated a sensor unit and a communication to the RFID-device for generating the continuously responding status data.. The inventive monitoring system also replace the huge amount of connecting wires of sensor units of different devices for analyzing temperature or air pressure values because of the short connection between RFID-device and sensor and the transmitting of the continuously responding status data independent of near or distance field technology. Furthermore the integration of sensor units offers the opportunity for having a clearly overview of data, which are normally controlled in the maintenance mode.

In a particular preferred development the continuously, responding status data of the RFID-device provides health and usage data. Providing data of quantity consumed is advantageous because of increasing the monitoring possibilities. Monitoring of the functionality of components and supply items gives a high process reliability of the system in an aircraft, monitoring the health and usage data increases safety reliability and the sequences reliability. Having a continuously overview of the oxygen system also for the oxygen mask improves the reliability of the system and increases the process stability for an emergency case. But also the lifetime of components can be adapted by determining the next tonus for replacing the used element. Further the passenger service having a complete assortment can be improved by getting continuously monitor information of the consumption of the convenience goods such as toilet paper, soap. refreshingly cooled drinks, but also oxygen amount and so on.

In a particular preferred development the continuously, responding status data are provided in a defined time interval. During the flight it is not necessary for all cabin elements to provide a continuously, responding status data, but only for certain specific elements. Other cabin elements like durable consumer goods can be monitored in a defined time interval to decrease the amount of data during flight and the energy input.

In a particular preferred development the continuously, responding status data are provided after an upper and/or lower warning limit. For a further decreasing the amount of data and the energy input for non-durable consumer goods an upper or lower warning limit can initiate the monitor signal to the RFID-reader analyzed in a continuously responding status. Further the upper and lower warning limit can be used as a warning signal for the stand-by-duty of supply items.

In a particular preferred development the continuously, responding status data are adapted for maintenance modus. In the maintenance modus all elements, components and supply item in a cabin or fuselage will be tracked, just as the whole aircraft proofing the security and functionality of each part. For replacing the elaborate hand-held reader in maintenance mode the serviceable development is provided also in the maintenance mode on the ground of the airport and can also track the history of each aircraft cabin element during flight. The continuously responding status data offers the opportunity to see changes in the functionality of the element which can be observed during flight but also be tracked in the maintenance mode to get a lifetime overview of the all elements.

In a particular preferred development the continuously, responding status data are provided to a hand held readers. In case of recheck the integrated RFID-reader getting a double check monitoring an additional hand held reader can offer the continuously, responding status data for comparing and ratifying the monitoring.

In a particular preferred development the operating range of the configuration and monitoring system for continuously, responding status data is extended to the fuselage of an aircraft. This development considers the overlap and / or intersection of the cabin elements, components and supply items with the fuselage and integrates the monitoring in the master plan of the aircraft. Further the benefit for tracking the history of each aircraft cabin element during flight is also important for the whole fuselage and not only for the part of it.

In a particular preferred development the continuously, responding status data are provided powerless, preferably using energy harvesting system instead of a power source or power reservoir or in combination with a power reservoir like a battery or capacitor or in combination with a power source. The known near field devices-also referred to a as passive RFID-devices-- offer the possibility to get continuously responding status data gaining their energy from establishing an inductive field from the radio signals of the scanners. The missing source of energy does result in lower ranges. The adapted repeater of the concept proposed above can expand the monitor signal to increase the scanning range; thus this is a prerequisite to allow to establish an on-board configuration and monitoring system for an aircraft cabin element of a plurality of cabin elements in a cabin of an aircraft, which as such is independent from service points at ground. Depending on the amount of the passive and active RFID-devices and on the amount of the repeater the configuration and monitoring system can lower the need energy of integrated power supply and therefore the energy consumption of an aircraft during flight which is necessary for continuously monitoring with a huge amount of status data of the RFID-devices.

In a particular preferred development an aircraft is conFig.d with at least a cabin and a cabin element from a plurality of cabin elements and the aircraft cabin element configuration with the monitor information includes continuously, responding status data. Therefore the monitoring is expanded to each cabin of an aircraft, also the cockpit wherein the monitoring signal provides data being needed to observe or to track.

In a particular preferred development a method of monitoring an aircraft cabin element is provided, comprising a radio frequency identification device (RFID), wherein the RFID-device is associated with the aircraft cabin element adapted for providing a monitor signal adapted to indicate a status of the aircraft cabin element by a monitor information, a RFID-reader communicatingly connectable to the RFID-device and a controlling unit of the cabin management system. The method comprises the steps of providing a monitor signal adapted to indicate a status of the aircraft cabin element by a monitor information communicating between the RFID and the RFID-reader, wherein the RFID-reader is communicatingly integrated in the central cabin system by being communicatingly connectable to the controlling unit in the cabin, wherein all information are centrally collectable and accessible by the controlling unit, expanding the monitor signal in the cabin to the RFID-reader by at least one repeater providing continuously responding status data of the aircraft cabin element during the flight in the monitor information. The advantageously development of the method offers a monitoring during flight getting useful information or status data which increases the projectable availability for use of each aircraft element.

In a particular preferred development the method of monitoring an aircraft further provides the connecting of RFID-reader for the continuously, responding status data is communicatingly to the controlling unit in the cabin for realising the continuously monitoring of the status data during flight readable and react able for the crew.

In a particular preferred development a method of monitoring an aircraft cabin element furthermore provide the centrally collecting and centrally accessing all information of the controlling unit of the continuously, responding status data.

For a more complete understanding of the invention, the invention will now be described in detail with reference to the accompanying drawing. The detailed description will illustrate and describe what is considered as a preferred embodiment of the invention; in particular with regard to an aircraft cabin element. It should of course be understood that various modifications and changes in form or detail could readily be made without departing from the spirit of the invention; in particular, as claimed, the inventive concept can also be realized for a fuselage element and/or an outside-cabin element alone or in combination with an aircraft cabin element. It is therefore intended that the invention may not be limited to the exact form and detail shown and described herein, nor to anything less than the whole of the invention disclosed herein and as claimed hereinafter. Further the features described in the description, the drawing and the claims disclosing the invention may be essential for the invention considered alone or in combination. In particular, any reference signs in the claims shall not be construed as limiting the scope of the invention. The wording "comprising" does not exclude other elements or steps. The wording "a" or "an" does not exclude a plurality. The wording, "a number of" items, comprises also the number one, i.e. a single item, and further numbers like two, three, four and so on.

The drawing shows in:
Fig. 1: an overview of a cabin of an aircraft integrating RFID-devices according to a first embodiment of a fuselage;
Fig 2: a simplified scheme of a configuration and monitoring system for cabin elements, aircraft components and supply items of a cabin and fuselage of an aircraft according to a second embodiment;
Fig. 3: a simplified scheme of the configuration and monitoring system for cabin elements, integrating sensor units in the RFID-devices according to a third embodiment;
Fig. 4: a detailed picture of the overhead panel integrating a repeater according to a forth embodiment;
Fig. 5: a simplified scheme for exemplifying a concept of integrating RFID-devices in a passenger seat according to a fifth embodiment.

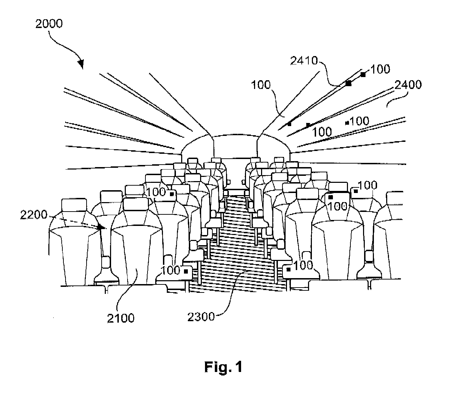

Fig. 1 shows an overview of a cabin 2000 of an aircraft. The section of the passengers' seats 2100 is divided by an alley, so called gangway 2300. The seats, parts of the cabin, are arranged in several seat rows 2200. Beyond the seats the overhead panel 2400, comprising a plurality of aircraft elements, is installed in the upper region of the cabin providing a lot of aircraft cabin elements, in particular aircraft cabin elements in combination with a functionality. An aircraft cabin element can be any item in an aircraft cabin without any functionality like a simple item of construction or the like; e.g. a piece of material whose status of maintenance is of interest. An aircraft cabin element is meant to comprise also an aircraft cabin element in combination with a functionality, which is refered to an aircraft cabin component. An aircraft cabin component can e.g. be an aircraft supply item like a reading light 2410. This can be split in more aircraft components and elements being monitored by configuration and monitoring system. The comprised RFID-devices 100 are associated to such an aircraft component like the reading light 2410 for providing a continuously responding status data.

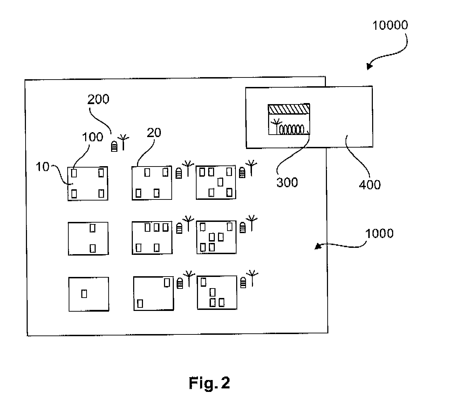

Fig. 2 shows a simplified scheme of the configuration and monitoring system 1000 for cabin elements, aircraft components and supply items of a cabin. A supply unit 10 integrates in this case four RFID-devices adapted for different aircraft elements, comprising a internal transponder and internal antenna, and communicating with the RFID-reader 300. The design and the modes function of the RFID-devices differ depending on the frequency range, just as with the antennas. An external repeater 200, which can be an AC antenna, expands the monitor signal of the RFID-devices providing a continuously responding status data during flight and radiates the electromagnetic waves generated by the reader. The RFID-reader 300 is integrated in the controlling unit 400 of the central cabin system 10000 signal connected with the controlling unit 400. The RFID-reader sends radio waves being detected by RFID-device and therefore activates the RFID-device or the repeater. The RFID-reader decodes the monitor signal of the continuously responding status in case of weak monitor signals with a low range of electromagnetic field depending of the reading ranges different types of readers are used for the low frequency, high frequency and ultra high frequency. The reading range can differ because of the position of the repeater and because of the frequency.

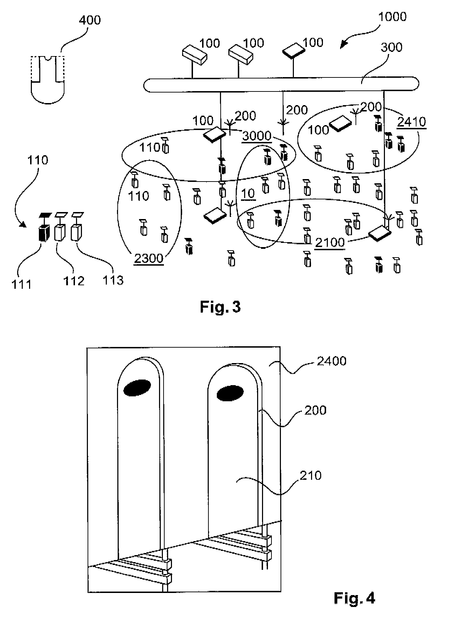

Fig. 3 shows a simplified scheme of the configuration and monitoring system for aircraft cabin elements and/or fuselage elements and/or outside-cabin elements --single, all or some of these kinds of elements-- having an RFID-device according to the above concept, in particular integrating a sensor unit in the RFID-device. A sensor is integrated in the RFID antenna to activate the RFID system 1000. In this embodiment one or more external sensor units 110 are assigned to the RFID-devices 100, in particular integrated in the RFID-device. In this Fig. 3 different combinations of the sensor units are marked with ovals, showing the distribution of different sensor units subdivided in three additional types of sensor units: temperature sensor unit 111, light sensor unit 112 und mechanical sensor unit 113. The drawn intersection shows that the sensor units are associated to different supply items or components, like the supply item for the fuselage which has intersections with the cabin and the gangway 2300. The cabin supply item 10 has also intersectional sensor unit adapted to the reading light 2410. But also separate performed supply items are possible like the passenger seat 2100. For the reading light supply item for instance an amount of sensor units, more precisely 1 temperature sensor unit 111, three lighting unit 112 and 1 mechanical sensor unit are associated to a RFID-device marked by the oval which is communicating with the RFD reader 300. This RFD reader communicates with different RFID-devices and is continuously connected the controlling unit 400.

Fig. 4 shows a overhead panel 2300 of a cabin wherein two repeater 200 are integrated in the overhead panel having a coil with windings. The integration of the repeater offers new possibility in existing components of an aircraft for realizing the configuration and monitoring system to built in near field RFID-devices without any external power supply in existing aircrafts because of the expanding of the monitor signal by the repeater for the provided continuously responding status data.

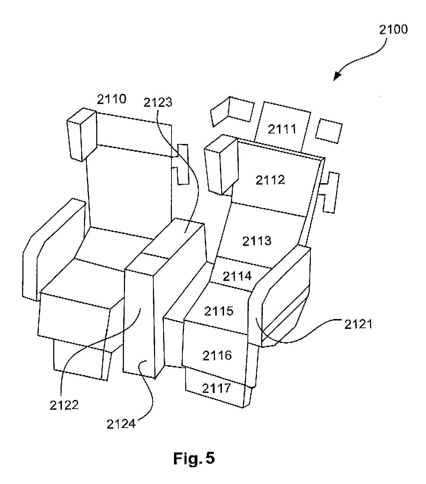

Fig. 5 shows a seat assembly 2100 of an aircraft with aircraft cabin elements in form of components and parts in which RFID-devices are integrated for lifetime and reliability issues. The seat assembly 2100 consist of several moveable seat position parts 2110, wherein the following components are movable, the head position component 2111, the shoulder position component 2112, the upper back component 2113, the lower back component with functional supply item 2114. The seating place component 2115 combined with the leg position component 2116 and foot position component 2117. Additional supply items are integrated in the arm position component 2120, the electronic regulation supply item setting the seat position, the mechanic supply item for the tablet position, the power socket supply item 2124 and the electronic supply item for the remote control 2123.

This Fig. 5 illustrates very the complexity of a single passenger seat being monitored which will be extremely more complex for the whole cabin. Therefore there is a need for getting continuously responding status data for monitoring the whole cabin. Furthermore beside a monitoring of the like aircraft cabin element in the fuselage a monitoring of a fuselage element and/or an outside-cabin element in the aircraft also during the flight for increasing the durability and reliability of each element, component and supply item of an aircraft is desirable.

**List of reference signs**

| | |
|---|---|
| 10 | cabin supply unit |
| 20 | component |
| 100 | RFID-device |
| 110 | sensor unit |
| 111 | temperature sensor unit |
| 112 | lighting sensor unit |
| 113 | mechanical sensor unit |
| 200 | repeater |
| 210 | coil with windings |
| 300 | RFID-reader |
| 400 | controlling unit |
| 1000 | aircraft element configuration and monitoring system |
| 2000 | cabin |
| 2100 | seat |
| 2110 | seat position parts |
| 2111 | head position component |
| 2112 | shoulder position component |
| 2113 | upper back component |
| 2114 | lower backcomponent with functional supply item |
| 2115 | seat place component |
| 2116 | leg position component |
| 2117 | foot position component |
| 2120 | arm position component |
| 2121 | electronic regulation supply item |
| 2122 | mechanic suplly item for tablet |
| 2123 | electronic supply item for remote control |
| 2124 | power socket supply item |
| 2200 | seat row |
| 2300 | gangway |
| 2400 | overhead panel |
| 2410 | reading light |
| 3000 | fuselage |
| 10000 | central cabin system |

## Claims

1. A configuration and monitoring system for an aircraft cabin element of a plurality of cabin elements in a cabin of an aircraft, the system comprising:
- a radio frequency identification (RFID) device, wherein the RFID-device is associated with the aircraft cabin element adapted for providing a monitor signal adapted to indicate a status of the aircraft cabin element by a monitor information,
- a RFID-reader communicatingly connectable to the RFID-device,
- a controlling unit of a central cabin system, **characterized in that**
- the RFID-reader is communicatingly integrated in the central cabin system by being continuously communicatingly connectable to the controlling unit in the cabin, wherein information are centrally collectable and accessable by the controlling unit, and
- at least one repeater is adapted for expanding the monitor signal in the cabin to the RFID-reader, wherein the monitor information includes continuously, responding status data of the aircraft cabin element during the flight.

2. A configuration and monitoring system according to claim 1, wherein the aircraft cabin element including the continuously, responding status data is adapted to a cabin component and/or a cabin supply item.

3. A configuration and monitoring system according to claim 1 or 2, wherein a RFID-device and a sensor unit is assigned to the RFID-device and/or the RFID-reader, in particular a sensor unit integrated in the RFID-device and/or the RFID-reader, communicating continuously, responding status data providing analyzable and transponder callable status data.

4. A configuration and monitoring system according to one of claims 1 to 3, wherein the continuously, responding status data of the RFID-device provides health and usage data.

5. A configuration and monitoring system according to one of claims 1 to 4, wherein the continuously, responding status data are provided in a defined time interval.

6. A configuration and monitoring system according to one of claims 1 to 5, wherein the continuously, responding status data are provided after upper and/or lower warning limit.

7. A configuration and monitoring system according to one of claims 1 to 6, wherein the continuously, responding status data are adapted for maintenance modus.

8. A configuration and monitoring system according to one of claims 1 to 7, wherein the continuously, responding status data are provided to a hand held readers.

9. A fuselage of an aircraft comprising a configuration and monitoring system according to one of claims 1 to 8, wherein the monitor information including the continuously, responding status is provided to an aircraft cabin element and/or a fuselage element.

10. An aircraft comprising a configuration and monitoring system according to one of claims 1 to 8, wherein the aircraft is conFig.d with at least a cabin and a cabin element from a plurality of cabin elements and the aircraft cabin element configuration with the monitor information includes the continuously, responding status data provided to provided to an aircraft cabin element and/or an outside-cabin element.

11. Method of monitoring an aircraft cabin element, comprising:
- a radio frequency identification device (RFID), wherein the RFID-device is associated with the aircraft cabin element adapted for providing a monitor signal adapted to indicate a status of the aircraft cabin element by a monitor information,
- a RFID-reader communicatingly connectable to the RFID-device,
- a controlling unit of the cabin management system, the method comprising the steps of:
- providing a monitor signal adapted to indicate a status of the aircraft cabin element by a monitor information
- communicating between the RFID-device and the RFID-reader, wherein the RFID-reader is communicatingly integrated in the central cabin system by being communicatingly connectable to the controlling unit in the cabin, wherein all information are centrally collectable and accessable by the controlling unit,
- expanding the monitor signal in the cabin to the RFID-reader by at least one repeater antenna,
- providing continuously responding status data of the aircraft cabin element during the flight in the monitor information.

12. Method of monitoring an aircraft cabin element according to claim 11, wherein the RFID-reader for the continuously, responding status data is communicatingly connecting to the controlling unit in the cabin.

13. Method of monitoring an aircraft cabin element according to claim 11 or 12, wherein the controlling unit is centrally collecting and accessing all information of the continuously, responding status data.
